# EUROPÄISCHE PATENTSCHRIFT

(11) **EP 0 943 599 B1**
(45) Veröffentlichungstag und Bekanntmachung des Hinweises auf die Patenterteilung: **26.12.2007**
(21) Anmeldenummer: 99104820.8
(22) Anmeldetag: 11.03.1999
(51) Int. Cl.: C07C 43/196, C10M 105/18, C11B 9/00

(54) **Neue Terpenether und deren Anwendungen**
Terpene ethers and their uses
Ethers terpéniques et leurs utilisations

(30) Priorität: 20.03.1998 DE 19812245
(43) Veröffentlichungstag der Anmeldung: 22.09.1999
(73) Patentinhaber: Clariant Produkte (Deutschland) GmbH, 65929 Frankfurt am Main (DE)
(72) Erfinder: Wagner, Adalbert Dr., 86456 Gablingen-Lützelburg (DE); Ebmeyer, Frank Dr., 21339 Lüneburg (DE); Stuhlmüller, Georg, 86456 Gablingen (DE); Simon, Maximilian Prof. Dr.-Ing., 82140 Olching (DE); Vojacek, Herbert Prof. Dr.-Ing, 83703 Gmund (DE)
(74) Vertreter: Luderschmidt, Wolfgang

(56) Entgegenhaltungen:
- EP-A- 0 082 967
- DE-A- 3 327 014
- US-A- 4 922 047
- US-A- 5 366 959
- S. KUCHARSKI: "Terpene ethers. Part VI. Synthesis of terpene ethers of polyethylene glycols" ROCZNIKI CHEMII, Bd. 45, Nr. 3, 1971, Seiten 479-484, XP002105709
- CHEMICAL ABSTRACTS, vol. 82, no. 18, 5. Mai 1975 Columbus, Ohio, US; abstract no. 113479z, M. YOSHIHARU: "Surfactants containing terpenyl group. VI. Synthesis and their surface activities of polyethylene glycol monoterpenyl ethers from various terpene hydrocarbons and polyethylene glycols" Seite 97; Spalte 1; XP002105710 & YUKAGAKU, Bd. 23, Nr. 12, 1974, Seiten 804-809, & "Chemical Abstracts Ninth Collective Index, Chemical Substances (Ethaneseleneno-F)" , AMERICAN CHEMICAL SOCIETY

## Beschreibung

Die Erfindung bezieht sich auf neue Terpenether, ein Verfahren zu ihrer Herstellung und ihre Verwendung u.a. als Traktionsfluide oder auf dem Gebiet der Riechstoffe und Lösungsmittel.

In geschmierten Traktionsgetrieben werden spezielle Kraftübertragungsfluide benötigt, die durch Reibung das Drehmoment des Antriebteils auf das Abtriebteil übertragen. Der Schmierfilm in der Berührungszone zwischen den beiden Wälzkörpern wird durch die übertragenen Reibkräfte auf Scherung beansprucht.

Das Anforderungsprofil für Traktionsfluide umfaßt u.a.
- gutes Tieftemperaturfließverhalten und ausreichende Viskosität bei Betriebstemperatur
- ausreichend hohes Reibzahlniveau über den Betriebstemperaturbereich
- geringe Verdampfungsverluste.

In EP-A-082 967 werden organische Verbindungen für die Verwendung als Flüssigkeit für Reibkraftübertragung beschrieben. In DE-A-3 321 773 und DE-A-3 337 503 sind cyclische Kohlenwasserstoffe zur Verwendung als Fluid für Zugantriebseinrichtungen beschrieben. DE 1 644 926 beschreibt kondensierte gesättigte Kohlenwasserstoffe als Mitziehflüssigkeit. Ferner sind in EP-A-319 580 Kohlenwasserstoffdiester zur Verwendung als Traktionsfluide aufgeführt. In DE-A-3 327 014 sind Terpenether beschrieben.

Unter anderem wird in DE-A-3 327 014 Beispiel 6 beschrieben (hier in Folge Vergleichsbeispiel 1 genannt). 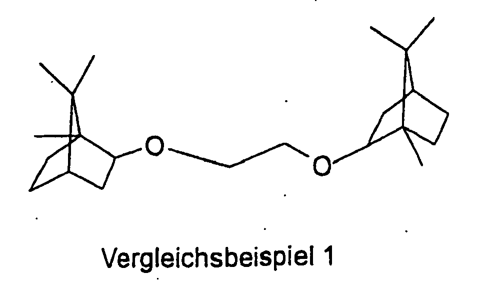 Vergleichsbeispiel 1 neigt bei längerem Stehen zur Kristallisation, weist einen Schmelzpunkt von 72 bis 75°C auf und erfüllt somit nicht die Anforderungen an gutes Tieftemperaturverhalten. Bei Verwendung als Traktionsfluid müssen die Erstarrungspunkte deutlich unter -20 °C liegen.

Aufgabe der vorliegenden Erfindung war somit, neue Verbindungen mit besserem Tieftemperaturverhalten zur Verfügung zu stellen.

Überraschenderweise wurde gefunden, daß bestimmte Terpenether trotz höherer Molekulargewichte dennoch deutlich erniedrigte Erstarrungspunkte aufweisen und somit das Kriterium Tieftemperaturfließfähigkeit erfüllen.

Gegenstand der Erfindung sind somit neue Terpenether gemäß Anspruch 1

Ebenso überraschend ist festgestellt worden, daß diese neuen Terpenether mindestens gleich hohes Reibzahlniveau, teilweise sogar höheres Reibzahlniveau aufweisen, als Vergleichsbeispiel 1. Dies widerspricht allen Erwartungen aufgrund systematischer Reibungsmessungen mit Modellsubstanzen (Lit.: Dokumentation des BMFT: Tribologie Bd.2, Hrsg. Springer Verlag Berlin, Heidelberg, New York, 1982, S.281-313). Danach wäre mit zunehmender Kettenlänge der Etherbrücke zwischen den Isobornylresten aufgrund der damit verbundenen Abnahme der sterischen Hinderung mit einer Abnahme des Reibungszahlniveaus bei elastohydrodynamischen Betriebsbedingungen zu rechnen.

Bei den Terpenethern gemäß der Erfindung - nach IUPAC-Nomenklatur 1,7,7-Trimethyl-bicyclo-[2.2.1]-hept-2-yl-ethern (= Isobornylethern) kann der 1,7,7-Trimethyl-bicyclo-[2.2.1]-hept-2-yl-Rest in der d-, l- oder vorzugsweise als Racemat und der Ether-Rest
in der Exo- und/oder Endoform vorliegen.

Die neuen Verbindungen sind nach an sich bekannten Methoden herstellbar.

Die bevorzugte Verfahrensweise zur Herstellung der neuen Ether geht von Camphen aus, das mit Dipropylenglykol umgesetzt wird. Bei der in Gegenwart saurer Katalysatoren ablaufenden Reaktion findet intermediär eine Wagner-Meerwein-Umlagerung des Camphens in eine Camphenzwischenstufe statt.

Die Synthese wird bei Temperaturen zwischen Raumtemperatur (20 °C) und 160 °C, vorzugsweise bei 50 bis 140 °C und insbesondere bei 70 bis 120 °C durchgeführt. Man kann die Reaktionspartner je nach gewünschtem Produkt in äquimolaren Mengen einsetzen oder auch mit einem Überschuß des einen oder anderen Partners arbeiten. Zur Synthese von Diisobornylethern erwies sich ein Überschuß an Camphen als vorteilhaft.

Als Katalysatoren dienen Mineralsäuren wie Schwefelsäure, Perchlorsäure, Phosphorsäure, Chlorsulfonsäure usw., starke organische Säuren wie p-Toluolsulfonsäure, Methansulfonsäure, Kampfer-10-sulfonsäure, saure Ionenaustauscher oder Friedel-Crafts-Katalysatoren wie Bortrifluorid und dessen Additionsprodukte (z.B. Etherate, Eisessigkomplex), Aluminiumchlorid, Zinkchlorid, PdCl₂, Pd(OAc)₂, SbCl₃, SbCl₅, YtCl₃, LaCl₃, Zeolithe und andere, in Mengen von 0,1 bis 10, vorzugsweise 0,5 bis 6 und insbesondere 1 bis 4 Gew.-% bezogen auf eingesetztes Camphen.

Die Umsetzung kann in Gegenwart oder in Abwesenheit von inerten Lösemitteln vorgenommen werden. Geeignete Lösemittel sind z.B. aliphatische Kohlenwasserstoffe wie Pentan, Hexan, Benzinfraktionen, Chloroform oder Tetrachlorkohlenstoff, aromatische Kohlenwasserstoffe wie Toluol, Xylol, Chlorbenzol, cycloaliphatische Kohlenwasserstoffe wie Cyclohexan, Cyclooctan oder Ether wie Dioxan, Dibutylether oder Ethylenglykoldimethylether. Besonders empfehlenswert ist die Arbeitsweise ohne Lösemittelzugabe.

Bei der Umsetzung können im allgemeinen alle Reaktionspartner in ihrer gesamten Menge inklusive Katalysator vorgelegt werden. In manchen Fällen läuft die Reaktion leicht exotherm ab, so daß es hier vorteilhafter ist, den Katalysator und den Alkohol vorzulegen und bei der gewünschten Temperatur das Camphen zuzugeben.

Als Alkohol, die mit Camphen umgesetzt wird, ist Dipropylenglykol, zu nennen.

Die Reinigung der Reaktionsprodukte erfolgt im allgemeinen nach der Entfernung des Katalysators (z.B. Wasserwäsche oder Neutralisation mittels Basen oder einfache Filtration) durch Destillation, für manche Anwendungszwecke ist jedoch eine Destillation nicht erforderlich. Eine weitere Reinigungsmöglichkeit besteht in der Umkristallisation aus geeigneten Lösungsmitteln.

Die bevorzugten Reaktionsprodukte stellen niedrigviskose bis hochviskose Flüssigkeiten dar, sie sind farblos bis schwach gelb gefärbt.

Die neuen Verbindungen zeichnen sich, wie schon erwähnt, überraschend dadurch aus, daß sie im Vergleich zum Vergleichsbeispiel 1 (Bsp. 6 aus DE-A-332 7014 A1) bis -30 °C nicht erstarren. Bei ihrer Verwendung als Traktionsfluide können die beschriebenen Verbindungen alleine oder als Gemische mit anderen Stoffen eingesetzt werden, wobei der Hauptanteil dieser Gemische aus einer oder mehreren der hier beschriebenen Verbindungen besteht.

Die neuen Verbindungen weisen z.T. ausgeprägten Riechstoffcharakter auf und sind daher für sich alleine als Duftstoffe oder auch in Duftstoffkombination, also in Gemischen mit synthetischen und natürlichen Ölen, Alkoholen, Aldehyden, Ketonen oder Estern einsetzbar, sie eignen sich ferner zur Parfümierung von Seifen, Detergentien, Pudern, Badeölen, Haarpflegemitteln, Cremes sowie weiteren bekannten, Duftstoff enthaltenden Zubereitungen. Ein größerer Teil der neuen Terpenether ist aufgrund seiner Konsistenz als Fixateur für Riechstoffe von Interesse, die dafür geeigneten Verbindungen haben als viskose Flüssigkeiten die Eigenschaft, die Flüchtigkeit von Riechstoffen stark herabzusetzen. Weiterhin lassen sich manche der Produkte als Lösemittel z.B. für Harze und Lacke, verwenden.

Schließlich sind solche Vertreter der neuen Ether, die freie Hydroxylgruppe aufweisen, als reaktive Zwischenprodukte, z.B. für die Synthese von Pflanzenschutz-, Schädlingsbekämpfungs- und Arzneimitteln, geeignet.

In Tabelle 1 sind die zur Erläuterung der Erfindung nach der unten aufgeführten allgemeinen Vorschrift hergestellten Verbindungen zusammengefaßt. Allgemeine Vorschrift zur Synthese von Terpenethern.

Zu 1 Mol des entsprechenden trockenen Alkohols gibt man Katalysator zu und erwärmt die Mischung auf 80 °C bis die Suspension rührbar wird und tropft nun geschmolzenes technisches Camphen zu. Anschließend rührt man 24 h bei 80 °C. Danach gibt man zur Zersetzung des Katalysators bei 80 °C Wasser zu, rührt 30 Min. und trennt danach die wäßrige Phase ab. Dieser Vorgang wird noch einmal wiederholt. Das nicht umgesetzte Camphen wird mittels Wasserdampfdestillation entfernt. Das Ölbad wird zu diesem Zweck auf 140 °C erhitzt. Das Kondensat wird mit GC analysiert. Der Rückstand wird zur Entfernung von Restwasser und niedrigsiedender Nebenprodukte bei einem Vakuum von 40 mbar auf 170 °C erhitzt. Nach Abklingen der Schaumbildung wird die Lösung durch ein Faltenfilter filtriert, wobei das Produkt, bestehend aus Mono - und Diisobornylether, als niedrigviskoses Öl anfällt. Durch anschließende Destillation können Mono- und Diether getrennt werden.

**Tabelle 1 Synthese von Terpenether**

| Alkohol | Produkt | Äquivalente Camphen | Katalysator (Äquivalente) | Sdp °C (Druck) | FP | Molare Masse** (M+H) |
|---|---|---|---|---|---|---|
| Ethylenglykol | Ethylenglykoldiisobornylether Vergleichsbeispiel 1 | 3* | AlCl3 (0.037) | 168 -169 (1,3 mbar) | 72-75 °C | - |
| 1,2-Dipropylenglykol | 1,2-Dipropylenglykolisobornylether Beispiel 3 | 4 | BF3-2CH3CO2H (0.037) | n.d. | <-30 °C | 271 |
| 1,2-Dipropylenglykol | 1,2-Dipropylenglykoldiisobomylether Beispiel 3a | 4 | BF3-2CH3CO2H (0.037) | 177 (0.4 bar) | < - 30 °C | 407 |

| | | | | | | |
|---|---|---|---|---|---|---|
| * Toluol als Lösungsmittel ** Massenspektrum DCI (Desorption Chemical Ionisation). n.d. nicht bestimmt, da Fraktion durch Nebenprodukte verunreinigt war | | | | | | |

### Tabelle 2: Reibzahlen von Modellsubstanzen variabler Kettenlänge

Die Reibungsmessungen wurden an einem Zweischeibenprüfstand durchgeführt, wie in der auf Seite 2 zitierten Literaturstelle beschrieben. Es wurden Mittelwerte der Reibungszahlen gegenübergestellt für folgende Grenzen der Betriebsparameter. Mittlere Hertsch Pressung pₘ = 500.....1260 N/mm²
Umfangsgeschwindigkeit v. = 0,42 .... 8,4 m/s
Schlupf s kleiner 6%
Temperatur T = 50°C
Rauheit der Reibkörperoberflächen entsprechend der von üblichen Reibradgetriebebauarten

| | Modellsubstanz | Mittlere Reibungszahl |
|---|---|---|
| A1 | 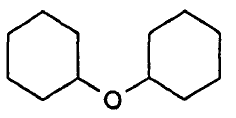 | 0,086 |
| A2 | 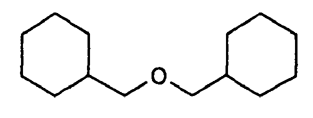 | 0,076 |
| A3 | 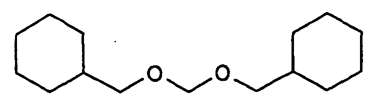 | 0,075 |
| B1 | 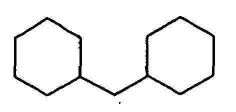 | 0,08 |
| B2 | 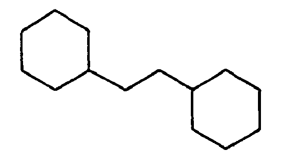 | 0,078 |
| C1 | 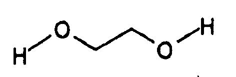 | 0,017 |
| C2 | 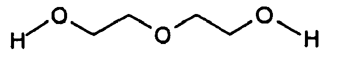 | 0,016 |
| C3 | 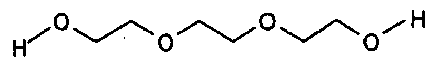 | 0,015 |
| Vergleichsbeispiel 1 | Ethylenglykoldiisobornylether | 0,094 |
| Bsp. 3a | Dipropylenglykoldiisobornylether | 0,099 |

## Patentansprüche

1. Terpenether umfassend 1,2-Dipropylenglykolisobornylether und 1,2-Dipropylenglykoldiisobomylether

2. Verfahren zur Herstellung von Terpenethern nach Anspruch 1, **dadurch gekennzeichnet, dass** Camphen mit Dipropylenglykol
unter Einsatz von sauren Katalysatoren umgesetzt wird.

3. Verwendung der Terpenether nach Anspruch 1 als Traktionsflüssigkeit in Traktionsgetrieben.

4. Verwendung gemäss Anspruch 3, **dadurch gekennzeichnet, dass** man eine der beanspruchten Verbindungen oder eine Mischung von zwei oder mehreren der beanspruchten Verbindungen einsetzt.

5. Verwendung gemäss Anspruch 4, **dadurch gekennzeichnet, dass** man die beanspruchten Verbindungen im Gemisch mit anderen Traktionsfluiden einsetzt.

6. Verwendung gemäss Anspruch 3, **dadurch gekennzeichnet, dass** man die beanspruchten Verbindungen in einer Konzentration von mindestens 5 Gew. %, insbesondere 20 bis 95 Gew.%, einsetzt.

7. Verwendung nach einem der Ansprüche 3 bis 6, **dadurch gekennzeichnet, dass** die Traktionsfluide übliche Additive enthalten.

8. Verwendung der Terpenether nach Anspruch 1 als Duftstoff in Kosmetika und Körperpflegemitteln.

## Claims

1. Terpene ether comprising 1,2-dipropylene glycol isobornyl ether and 1,2-dipropylene glycol diisobornyl ether.

2. A method for preparing terpene ethers according to Claim 1, **characterized in that** camphene is reacted with dipropylene glycol under the presence of acidic catalysts.

3. The use of the terpene ethers according to Claim 1 as traction fluid in traction gears.

4. The use according to Claim 3, **characterized in that** one of the claimed compounds or a mixture of two or more of the claimed compounds is used.

5. The use according to Claim 4, **characterized in that** the claimed compounds are used in a mixture with other traction fluids.

6. The use according to Claim 3, **characterized in that** the claimed compounds are used in a concentration of at least 5% by weight, in particular 20 to 95% by weight.

7. The use according to one of Claims 3 to 6, **characterized in that** the traction fluids comprise conventional additives.

8. The use of the terpene ethers according to Claim 1 as fragrance in cosmetics and body care products.

## Revendications

1. Ether terpénique comprenant l'éther 1,2-dipropylèneglycolisobornyle et l'éther 1,2-dipropylèneglycoldiisobornyle.

2. Procédé pour la fabrication d'éthers terpéniques selon la revendication 1, **caractérisé en ce qu'**on met dans la réaction du camphène avec du dipropylèneglycol par utilisation de catalyseurs acides.

3. Utilisation d'éther terpénique comme liquide de traction en engrenages de traction.

4. Utilisation selon la revendication 3, **caractérisée en ce qu'**on utilise l'un des composés revendiqués ou un mélange de deux ou plusieurs composés revendiqués.

5. Utilisation selon la revendication 4, **caractérisée en ce qu'**on utilise les composés revendiqués en mélange avec d'autres fluides de traction.

6. Utilisation selon la revendication 3, **caractérisée en ce qu'**on utilise les composés revendiqués avec une concentration d'au moins 5 pour cents en poids, spécialement 20 à 95 pour cents en poids.

7. Utilisation selon l'une des revendications 3 à 6, **caractérisée en ce que** les fluides de traction contiennent des additifs conventionnels.

8. Utilisation d'éthers terpéniques selon la revendication 1, comme des substances aromatiques dans les produits cosmétiques et les moyens de soin du corps.
